# EUROPEAN PATENT SPECIFICATION

(11) **EP 3 424 353 B1**
(45) Date of publication and mention of the grant of the patent: **28.04.2021**
(21) Application number: 18202992.6
(22) Date of filing: 26.10.2018
(51) Int. Cl.: A24F 40/465

(54) **VAPORIZER AND LOW-TEMPERATURE SMOKING SET**
VERDAMPFER UND NIEDRIGTEMPERATUR RAUCHVORRICHTUNG
VAPORISATEUR ET ENSEMBLE À FUMER COMBINÉ À BASSE TEMPÉRATURE

(30) Priority: 27.10.2017 CN 201721428282 U
(43) Date of publication of application: 09.01.2019
(73) Proprietor: Shenzhen First Union Technology Co., Ltd., 518103 Shenzhen, Guangdong (CN)
(72) Inventor: WU, Zexin, Shenzhen, Guangdong 518103 (CN); LI, Yonghai, Shenzhen, Guangdong 518103 (CN); XU, Zhongli, Shenzhen, Guangdong 518103 (CN)
(74) Representative: Proi World Intellectual Property GmbH

(56) References cited:
- WO-A1-95/27411
- WO-A1-2016/023809
- CN-U- 206 227 716
- US-A1- 2016 120 221
- US-A1- 2017 224 015

## Description

### TECHNICAL FIELD

The present disclosure relates to the field of smoking sets, and particularly, to a vaporizer and a low-temperature baked smoking set having same From CN-206 227 716 U is a prior art vaporizer known.

### BACKGROUND ART

With the increasing amount of smoking people in our country, the tobacco cigarettes are applied all over the world. It is difficult to avoid the second hand smoking. The traditional cigarettes need to be lit by open flame to generate smoking smog. The tobacco cigarettes release lots of noxious materials to human people during high-temperature combustion. Most smokers already know the hazard of smoking, but can't control themselves, it is extremely difficult to get out of smoking.

Recently, a low-temperature baked smoking set mainly use some solid vaporizable materials such as tobacco shreds or opium paste etc. to be baked at a low-temperature to generate smoking smog for inhaling, which tremendously reduces the noxious materials to produce. The low-temperature baked smoking set generally includes a vaporizer for receiving and heating the tobacco cigarettes to generate smoking smog.
In the present disclosure, the inventors found the relative technologies of aforementioned have blow problems: in a process of the vaporizer being disposable heating the tobacco cigarette, the flavor and taste of smoking will be weakened at later stage, which causes different flavors at earlier and later stages to influence user experience.

### SUMMARY

In view of the drawbacks in the prior art, the present disclosure relates to a vaporizer and a low-temperature baked smoking set have the same.

In order to solve the above technical problem, the present disclosure provides a low-temperature baked vaporizer according to independent claim 1 whereas various embodiments of the vaporizer and improvements thereto are recited in the dependent claims. The vaporizer includes the features set out in claim 1.

Preferably, the sleeve is a hollow cylinder, the first sleeve has same inner diameter as the second sleeve.

Preferably, the electromagnetic induction coil encircles outside the first sleeve, configured for heating the sleeve.

Preferably, a number of turns of the electromagnetic induction coil is in accordance with a length of the first sleeve.

Preferably, the first sleeve and the second sleeve are made from same or different kinds of metallic materials.

Preferably, the first sleeve and the second sleeve are made from different metallic magnet materials; the first sleeve has a bigger magnetic permeability than the second sleeve.

Preferably, an insulating layer encircles outside the sleeve, configured for making less heat of the sleeve delivered to outside.

Preferably, the sleeve has a tobacco receptacle for receiving the solid tobacco materials.

Preferably, the vaporizer further includes a power supply module, coupled with the electromagnetic induction coil, and configured for supplying power to the electromagnetic induction coil.

Preferably, the electromagnetic induction coil includes a first interface and a second interface; the first interface and the second interface are coupled with the power supply module.

Preferably, an insulating layer is coated to the electromagnetic induction coil, configured for preventing leakage of electricity of the electromagnetic induction coil.

Preferably, the power supply module includes an USB interface, a battery, a control unit, a charging circuit, a discharging circuit, a voltage detecting circuit, two switches, a battery management circuit and a converter; the battery is coupled with the charging circuit and discharging circuit, two switches are respectively disposed between the battery and the charging circuit, and between the battery and the discharging circuit. The charging circuit and discharging circuit are both coupled with the USB interface. The discharging circuit is coupled with the battery management circuit. The battery management circuit is coupled with the converter. The converter is coupled with the electromagnetic induction coil. The voltage detecting circuit is coupled with the USB interface. The control unit is coupled with the two switches and the voltage detecting circuit.

To solve the above problem, the present disclosure further provides a low-temperature baked smoking set having a housing and the aforementioned vaporizer; the vaporizer is disposed inside the housing.

Additional aspects and advantages of the present disclosure will be: by relying on the vaporizer of the low-temperature baked smoking set, it includes the electromagnetic induction coil and the sleeve for receiving vaporizable materials; the sleeve is made from metallic materials, the electromagnetic induction coil encircles outside the sleeve, the sleeve includes a first sleeve and a second sleeve connected with the first sleeve, the cross-sectional area of the first sleeve is smaller than that of the second sleeve along the transverse axis, and the second sleeve has a bigger mass than the first sleeve such that increasing rate of temperature of the second sleeve is slower than that of the first sleeve, which may realize segment heating of the vaporizer, enable consistent flavor during smoking, improve user experience and reduce the cost.

### BRIEF DESCRIPTION OF THE DRAWINGS

Many aspects of the present disclosure can be better understood with reference to the following drawings. The components in the drawings are not necessarily drawn to scale, the emphasis instead being placed upon clearly illustrating the principles of the present disclosure. Moreover, in the drawings, like reference numerals designate corresponding parts throughout the several views.
FIG. 1 is an aspect view of a low-temperature baked smoking set in accordance with one embodiment of the present disclosure;
FIG. 2 is an isometric view of a vaporizer of the low-temperature baked smoking set in FIG. 1.
FIG. 3 is an isometric view of a sleeve of the low-temperature baked vaporizer in FIG. 1.
FIG. 4 is an isometric view of an electromagnetic induction coil of the low-temperature baked vaporizer in FIG. 1.
FIG.5 is a block diagram of a power supply module of the low-temperature baked vaporizer in FIG. 1.

### DETAILED DESCRIPTION

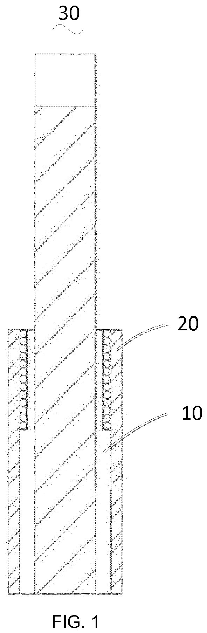

The structure and operating principle of the above vaporizer and the low-temperature baked smoking set are illustrated below, mainly shown from FIG. 1 to FIG. 5 in further detail using exemplary embodiments.

Referring to FIG. 1, which is an aspect view of a low-temperature baked smoking set in accordance with one embodiment of the present disclosure; The low-temperature baked smoking set 30 includes a vaporizer 10 and a housing 20. The vaporizer 10a is accommodated inside the housing 20.

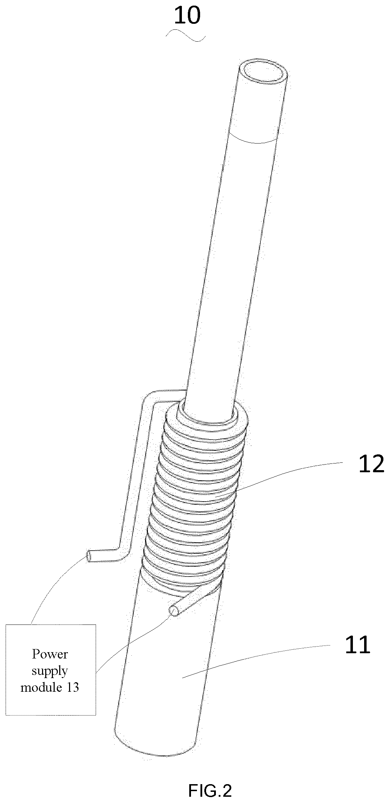

Referring to FIG. 2, the vaporizer 10 includes a sleeve 11, an electromagnetic induction coil 12 and a power supply 13. The sleeve 11 is configured for receiving solid vaporizable materials; the electromagnetic induction coil 12 encircles outside the sleeve 11 for heating the sleeve 11. The power supply module 13 is coupled with the electromagnetic induction coil 12 to output alternative current to generate an induction current.

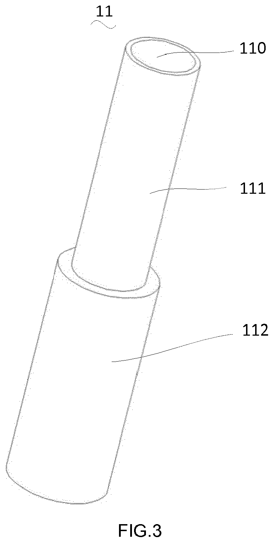

Referring to FIG. 3, the sleeve 11 is a hollow cylinder, the sleeve 11 is made from metallic materials, preferably magnetic materials to make the effect of inductive heating better, such as powdered iron core, FeNI50 alloys, FeSiAl alloys and FeNiMo alloys etc. The sleeve 11 includes a first sleeve 111 and a second sleeve 112 connected with the first sleeve 111; the second sleeve 112 has a bigger mass than the first sleeve 111 such that increasing rate of temperature of the second sleeve is slower than that of the first sleeve. For example, a longitudinal axis extending along a same axis direction of the first sleeve 111 and the second sleeve 112, and a transverse axis that is perpendicular to and shorter that the longitudinal axis; a cross-sectional area of the first sleeve 111 is equal with that of the second sleeve 112 along the transverse axis, and a length of the first sleeve 111 is shorter than that of the second sleeve112 such that the second sleeve 112 has a bigger mass than the first sleeve 111. Or, the cross-sectional area of the first sleeve 111 is smaller than that of the second sleeve 112 along the transverse axis, the length of the first sleeve 111 is the same as that of the second sleeve112 such that the second sleeve 112 has a bigger mass than the first sleeve 111. Or, the cross-sectional area of the first sleeve 111 is smaller than that of the second sleeve 112 along the transverse axis, the length of the first sleeve 111 is shorter than that of the second sleeve112 such that the second sleeve 112 has a bigger mass than the first sleeve 111. Preferably, the cross-sectional area of the first sleeve 111 is smaller than that of the second sleeve 112 along the transverse axis, and inner diameter of the first sleeve 111 is equal with that of the second sleeve 112, the outer diameter of the first sleeve 111 is less than that of the second sleeve 112, let the solid vaporizable materials abut against inside of the sleeve 11

The sleeve 11 is heated by induction heating of the electromagnetic induction coil 12, since the cross-sectional area of the first sleeve 111 is smaller than that of the second sleeve 112 along the transverse axis, and the second sleeve 112 has a bigger mass than the first sleeve 111, according to a heat quantity calculation formula: Q = c * m *ΔT, of which, c is the specific heat capacity, m is the mass, ΔT is the changing temperature and Q is the heat quantity; and a power calculation formula: P = W/t, of which, P is the electric power, W is the electric work, t is the time, presuming the energy conversion efficiency η is Q = W * η, then P * t = c * m *Δ T * η, therefore, when P is a constant, objects with same materials that have same specific heat capacity, if the object has bigger mass m, then the object need more time to rise to a same temperature. Therefore, during heating the sleeve11, since the second sleeve 112 has a bigger mass than the first sleeve 111, the first sleeve 11 needs less time to rise to a preset temperature, but the second sleeve 112 needs more time to rise to the preset temperature, which may realize the segment heating.

An insulating layer (not shown) encircles outside the sleeve 11, configured for making less heat of the sleeve 11delivered to outside. The insulating layer is made from thermal insulation materials, such as, thermal insulation glue, aerogel, asbestos, aluminum silicate and calcium silicate etc. The insulating layer may further improve thermal efficiency and heat preservation effect, making the heating rate faster.

The sleeve 11 has a tobacco receptacle 110 for receiving the solid tobacco materials. For example, the solid tobacco materials is at least one or more selected from a group of tobacco shreds, tobacco sheets, tobacco powders and tobacco rods.

In some embodiment, it makes sense that the first sleeve 111 and second sleeve 112 are both made from same metallic magnetic materials or different metallic magnetic materials, such as the first sleeve 111 and the second sleeve 112 are both made from a conductor A, or the first sleeve 111 is made from the conductor A and the second sleeve 112 is made from a conductor B. When the first sleeve 111 and the second sleeve 112 are made from different metallic magnetic materials, the first sleeve 111 has a larger magnetic permeability than the second sleeve112, so when the electromagnetic induction coil 12 heats the sleeve 11, the first sleeve 111 generates larger amount of heat than the second sleeve 112, therefore realizing segment heating.

In some embodiment, it makes sense that the cross-section of the sleeve 11 is shaped as square, rectangular or irregular quadrilateral etc. just to guarantee that the sleeve 11 is able to receive the solid vaporizable materials and the second sleeve 112 has a bigger mass than the first sleeve 111.

In some embodiment, the sleeve 111 is a hollow body, the cross-sectional area refers to entity part of the body, such as, if the sleeve 11 is a hollow cylinder, the cross-section is a annular, so cross-sectional area is the area of the annular.

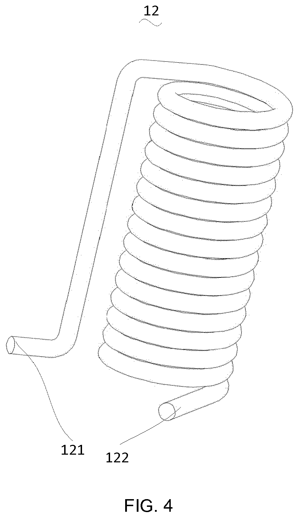

Referring to FIG. 4, the above electromagnetic induction coil 12 encircles outside the sleeve 11 following an unified direction, to exert inductive heating around the sleeve 11. The electromagnetic induction coil 12 may be made by electric-conductive materials, such as copper wires or aluminum wires etc. with good electric-conduction but cheap price. Preferably, the electromagnetic induction coil 12 encircles outside the first sleeve 111 to heat the first sleeve 111. A number of turns of the electromagnetic induction coil 12 is in accordance with a length of the first sleeve 111. When the electromagnetic induction coil 12 is electrified, by relying on the inductive heating, the temperature of the first sleeve 111 is risen soon, meanwhile, since the electromagnetic induction coil 12 encircles outside the first sleeve 111, the temperature of the second sleeve 112 is risen by heat conductivity from the first sleeve 111. If the heat of conductivity is constant, the first sleeve 111 has a smaller cross-sectional area and a smaller mass than the second sleeve 112, but in same material, after the heat conductivity is finished, based on the formula: Q = c * m *Δ T, of which, c is the specific heat capacity, m is the mass, Δ T is the changing temperature and Q is the heat quantity; if Q is the constant, when c is the constant, larger m, then Δ T is more slighter. So at a certain moment, the temperature of the second sleeve 112 is permanently lower than that of the first sleeve 111, with consequently realizing segment heating. For example, when the first sleeve 111 is heated to 30°C, the temperature of the second sleeve 112 won't be risen to 30°C until 30 seconds later; when the first sleeve 111 is continuously heated to 40°C, after the 30 seconds, the temperature of the second sleeve 112 won't be risen to 40°C until 10 seconds later.

The electromagnetic induction coil 12 includes a first interface 121 and a second interface 122; the first interface 121 and the second interface 122 are coupled with the power supply module 13 configured for supplying power to the electromagnetic induction coil 12, so the electromagnetic induction coil 12 generates the electromagnetic induction. Based on the electromagnetic induction law, when the electromagnetic induction coil 12 is electrified with the alternative current, the electromagnetic induction coil 12 generates alternative magnetic flux inside, the alternative magnetic flux generates induced potential inside the sleeve 11, meanwhile when magnetic lines of force in the magnetic field passes through the sleeve 11, the alternative magnetic lines of force forms a loop inside the sleeve 11, and the cross-sectional area of the sleeve 11 generates the inductive current, that is vortex, for heating the sleeve 11.

In some embodiment, it makes sense that the electromagnetic induction coil 12 is able to extend into the tobacco receptacle 110, or be embedded inside of the first sleeve 111, just to guarantee that the first sleeve 111is inducted by the magnetic field to generate heat. In other embodiments, the number of the electromagnetic induction coils 12 is two, including a first coil and a second coil (not shown). The first coil and the second coil both encircle outside the first sleeve 111, the first coil and the second coil may have different turns of coil to change the heating rate of the sleeve 11, which is more flexible to adjust the heating effect.

In some embodiment, it makes sense that the number of turns of the electromagnetic induction coils 12 may be more than or less than the length of the first sleeve 111, the number may be determined by the cross-sectional area of the electromagnetic induction coils 12 and the cross-sectional area of the first sleeve 111.

In some embodiment, it makes sense that an insulating layer is coated to the electromagnetic induction coil 12, configured for preventing leakage of electricity of the electromagnetic induction coil 12. The insulating layer is made by insulating materials such as, synthetic resin, epoxy resin, phenolic resin, 4250-plastic and polyimide plastic etc.

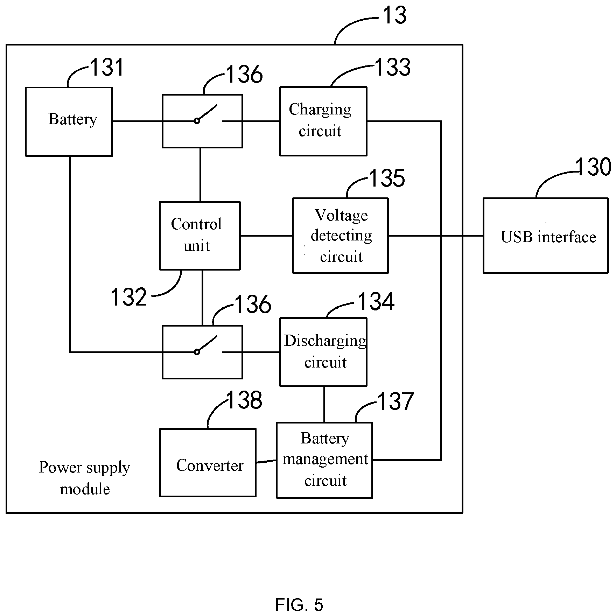

Referring to FIG.5, the above power supply module 13 is coupled with the first interface 121 and the second interface respectively, that means coupled with the electromagnetic induction coil 12. The power supply module 13 includes an USB interface 130, a battery 131, a control unit 132, a charging circuit 133, a discharging circuit 134, a voltage detecting circuit 135, two switches 136, a battery management circuit 137 and a converter 138; the battery 131 is coupled with the charging circuit 133 and discharging circuit 134, two switches 136 are respectively disposed between the battery 131 and the charging circuit 133, and between the battery 131 and the discharging circuit 134. The charging circuit 133 and discharging circuit 134 are both coupled with the USB interface 130 to be coupled with the external power supply. The voltage detecting circuit 135 is coupled with the USB interface 130, for detecting weather the power supply module 13 is coupled with the external power supply. The control unit 132 is coupled with the two switches 136 and the voltage detecting circuit 135. The discharging circuit 134 is coupled with the battery management circuit 137. The battery management circuit 137 is coupled with the converter 138 configured for direct current conversed to alternative current. The converter 138 is coupled with the electromagnetic induction coil 12 for supplying power to the electromagnetic induction coil 12. If the voltage detecting circuit 135 detects a voltage, that means the power supply module 13 is coupled with external power supply, then an electrical signal is sent to the control unit 132, after receiving the electrical signal, the control unit 132 controls the switch 136 between the battery 131 and the charging circuit 133 from "off' state alternative to "on" state, the external power supply supplies power to the battery 131 by current from the external power supply passing the charging circuit 133. If the voltage detecting circuit 135 fails to detect a voltage, that means, the power supply module 13 fails to be electrified with the external power supply, the control unit 132 generates another electrical signal, after the control unit 132 receives the electrical signal, the control unit 132 controls another switch 136 between the battery 131 and the discharge circuit 134 from "off' state alternative to "on" state. The current of the battery flows towards the converter 138 via the discharging circuit 134 and the battery management circuit 137. The converter 138 may convert the direct current into the alternative current, therefore supplying power to the electromagnetic induction coil 12.

In terms of the vaporizer 10 in the present disclosure, the second sleeve 112 has a bigger mass than the first sleeve 111, a cross-sectional area of the first sleeve 111 is smaller than that of the second sleeve 112 along the transverse axis, that means, that increasing rate of temperature of the second sleeve 112 is slower than that of the first sleeve 111, therefore, the segment heating of the vaporizer 10 is realized, enabling consistent flavor during smoking, improving user experience and reducing the cost.

In the embodiments, the sleeve 11 is a nearly hollow cylinder, for receiving the vaporizer 10. The housing 20 may be a plastic housing, made of some heat preservation materials such as polycarbonate, polyurethane and polyimide etc. In other embodiments, the housing 20 may be made of metallic, coating plastic film on the housing 20 to achieve the heat preservation effect.

The low-temperature baked smoking set 30 in accordance with the embodiments of the present disclosure, includes the vaporizer 10 available of segment heating that makes the flavor consistent during being inhaled, effectively improves the user experience. Compared with the resistance heating in the prior art, it has lower price.

Terminology used herein is for the purpose of describing particular embodiments only and is not intended to be limiting of the invention.

## Claims

1. A low-temperature vaporizer (10), comprising:
an electromagnetic induction coil (12) and a sleeve (11), configured for receiving solid vaporizable materials; the sleeve is made from metallic materials;
the electromagnetic induction coil encircles the outside of the sleeve;
**characterised in that** the sleeve comprises a first sleeve (111) and a second sleeve (112) connected with the first sleeve;
a longitudinal axis extending along a same axis direction of the first sleeve and the second sleeve, and a transverse axis that is perpendicular to the longitudinal axis; a cross-sectional area of the first sleeve is smaller than that of the second sleeve along the transverse axis, and the second sleeve has a bigger mass than the first sleeve such during electromagnetic induction by the induction coil the increasing rate of temperature of the second sleeve is slower than that of the first sleeve.

2. The vaporizer according to claim 1, **characterized in that**, the sleeve is a hollow cylinder, the first sleeve has same inner diameter as the second sleeve.

3. The vaporizer according to claim 1, **characterized in that**, the electromagnetic induction coil encircles the outside of the first sleeve and is configured for heating the sleeve.

4. The vaporizer according to claim 3, **characterized in that**, the number of turns of the electromagnetic induction coil is in accordance with a length of the first sleeve.

5. The vaporizer according to claim 1, **characterized in that**, the first sleeve and the second sleeve are made from same or different kinds of metallic materials.

6. The vaporizer according to claim 5, wherein when first sleeve and the second sleeve are made from different metallic magnet materials; the first sleeve has a bigger magnetic permeability than the second sleeve.

7. The vaporizer according to claim 1, **characterized in that**, an insulating layer encircles outside the sleeve, configured for making less heat of the sleeve delivered to outside.

8. The vaporizer according to claim 1, **characterized in that**, the sleeve comprises a tobacco receptacle for receiving the solid tobacco materials.

9. The vaporizer according to any one of claims 1 to 8, **characterized in that**, the vaporizer further comprises a power supply module, coupled with the electromagnetic induction coil, and configured for supplying power to the electromagnetic induction coil.

10. The vaporizer according to claim 9, **characterized in that**, the electromagnetic induction coil comprises a first interface and a second interface; the first interface and the second interface are coupled with the power supply module.

11. The vaporizer according to claim 10, **characterized in that**, an insulating layer is coated to the electromagnetic induction coil, configured for preventing leakage of electricity of the electromagnetic induction coil.

12. The vaporizer according to claim 11, **characterized in that**, the power supply module comprises an USB interface, a battery, a control unit, a charging circuit, a discharging circuit, a voltage detecting circuit, two switches, a battery management circuit and a converter; the battery is coupled with the charging circuit and discharging circuit, two switches are respectively disposed between the battery and the charging circuit, and between the battery and the discharging circuit; the charging circuit and discharging circuit are both coupled with the USB interface; the discharging circuit is coupled with the battery management circuit; the battery management circuit is coupled with the converter; the converter is coupled with the electromagnetic induction coil, the voltage detecting circuit is coupled with the USB interface; the control unit is coupled with the two switches and the voltage detecting circuit.

13. A low-temperature baked smoking set (30), comprising a housing (20) and the vaporizer according to any one of claims 1 to 12; the vaporizer is disposed inside the housing.

## Patentansprüche

1. Niedertemperatur-Verdampfer (10), umfassend:
eine elektromagnetische Induktionsspule (12) und eine Hülse (11), die zur Aufnahme von festen verdampfbaren Materialien konfiguriert ist; die Hülse ist aus metallischen Materialien hergestellt;
die elektromagnetische Induktionsspule die Außenseite der Hülse umgibt;
**dadurch gekennzeichnet, dass**
die Hülse eine erste Hülse (111) und eine zweite Hülse (112) umfasst, die mit der ersten Hülse verbunden ist, eine Längsachse, die sich entlang einer gleichen Achsenrichtung der ersten Hülse und der zweiten Hülse erstreckt, und eine Querachse, die senkrecht zu der Längsachse ist; eine Querschnittsfläche der ersten Hülse kleiner als die der zweiten Hülse entlang der Querachse ist und die zweite Hülse eine größere Masse als die erste Hülse hat, so dass während der elektromagnetischen Induktion durch die Induktionsspule die Temperaturanstiegsrate der zweiten Hülse langsamer ist als die der ersten Hülse.

2. Verdampfer nach Anspruch 1, **dadurch gekennzeichnet, dass** die Hülse ein Hohlzylinder ist, die erste Hülse den gleichen Innendurchmesser wie die zweite Hülse hat.

3. Verdampfer nach Anspruch 1, **dadurch gekennzeichnet, dass** die elektromagnetische Induktionsspule die Außenseite der ersten Hülse umgibt und zur Erwärmung der Hülse ausgebildet ist.

4. Verdampfer nach Anspruch 3, **dadurch gekennzeichnet, dass** die Anzahl der Windungen der elektromagnetischen Induktionsspule mit einer Länge der ersten Hülse übereinstimmt.

5. Verdampfer nach Anspruch 1, **dadurch gekennzeichnet, dass** die erste Hülse und die zweite Hülse aus gleichen oder unterschiedlichen Arten von metallischen Materialien hergestellt sind.

6. Verdampfer nach Anspruch 5, **dadurch gekennzeichnet, dass**, wenn die erste Hülse und die zweite Hülse aus unterschiedlichen metallischen Magnetmaterialien hergestellt sind, die erste Hülse eine größere magnetische Permeabilität als die zweite Hülse aufweist.

7. Verdampfer nach Anspruch 1, **dadurch gekennzeichnet, dass** eine Isolierschicht die Außenseite der Hülse umgibt, die so gestaltet ist, dass weniger Wärme der Hülse nach außen abgegeben wird.

8. Vaporisator nach Anspruch 1, **dadurch gekennzeichnet, dass** die Hülse einen Tabakbehälter zur Aufnahme der festen Tabakmaterialien aufweist.

9. Vaporisator nach einem der Ansprüche 1 bis 8, **dadurch gekennzeichnet, dass** der Vaporisator ferner ein Stromversorgungsmodul umfasst, das mit der elektromagnetischen Induktionsspule gekoppelt und zur Stromversorgung der elektromagnetischen Induktionsspule konfiguriert ist.

10. Verdampfer nach Anspruch 9, **dadurch gekennzeichnet, dass** die elektromagnetische Induktionsspule eine erste Schnittstelle und eine zweite Schnittstelle umfasst; die erste Schnittstelle und die zweite Schnittstelle sind mit dem Stromversorgungsmodul gekoppelt.

11. Verdampfer nach Anspruch 10, **dadurch gekennzeichnet, dass** die elektromagnetische Induktionsspule mit einer Isolierschicht beschichtet ist, die so konfiguriert ist, dass sie ein Austreten von Elektrizität aus der elektromagnetischen Induktionsspule verhindert.

12. Verdampfer nach Anspruch 11, **dadurch gekennzeichnet, dass** das Stromversorgungsmodul eine USB-Schnittstelle, eine Batterie, eine Steuereinheit, eine Ladeschaltung, eine Entladeschaltung, eine Spannungserfassungsschaltung, zwei Schalter, eine Batterieverwaltungsschaltung und einen Wandler umfasst; die Batterie mit der Ladeschaltung und der Entladeschaltung gekoppelt ist, zwei Schalter jeweils zwischen der Batterie und der Ladeschaltung und zwischen der Batterie und der Entladeschaltung angeordnet sind; die Ladeschaltung und die Entladeschaltung sind beide mit der USB-Schnittstelle gekoppelt; die Entladeschaltung ist mit der Batterieverwaltungsschaltung gekoppelt; die Batterieverwaltungsschaltung ist mit dem Wandler gekoppelt; der Wandler ist mit der elektromagnetischen Induktionsspule gekoppelt, die Spannungserfassungsschaltung ist mit der USB-Schnittstelle gekoppelt; die Steuereinheit ist mit den beiden Schaltern und der Spannungserfassungsschaltung gekoppelt.

13. Niedertemperatur-Backräucherset (30), umfassend ein Gehäuse (20) und den Verdampfer nach einem der Ansprüche 1 bis 12; der Verdampfer ist innerhalb des Gehäuses angeordnet.

## Revendications

1. Un vaporisateur à basse température (10) comprenant :
une bobine d'induction électromagnétique (12) et un manchon (11), configuré pour recevoir des matériaux vaporisables solides ; le manchon est fait de matériaux métalliques ;
la bobine d'induction électromagnétique encercle l'extérieur du manchon ;
**caractérisé en ce que**
le manchon comprend un premier manchon (111) et un second manchon (112) relié au premier manchon ; un axe longitudinal s'étendant le long d'une même direction axiale du premier manchon et du second manchon, et un axe transversal qui est perpendiculaire à l'axe longitudinal ; une surface de section transversale du premier manchon est plus petite que celle du second manchon le long de l'axe transversal, et
le second manchon a une masse plus importante que le manchon first de sorte que pendant l'induction électromagnétique par la bobine d'induction, la vitesse d'augmentation de la température du second manchon est plus lente que celle du premier manchon.

2. Vaporisateur selon la revendication 1, **caractérisé en ce que**, le manchon est un cylindre creux, le premier manchon a le même diamètre intérieur que le second manchon.

3. Vaporisateur selon la revendication 1, **caractérisé en ce que** la bobine d'induction électromagnétique encercle l'extérieur du premier manchon et est configurée pour chauffer le manchon.

4. Vaporisateur selon la revendication 3, **caractérisé en ce que** le nombre de tours de la bobine d'induction électromagnétique est conforme à une longueur du premier manchon.

5. Vaporisateur selon la revendication 1, **caractérisé en ce que** le premier manchon et le second manchon sont fabriqués à partir de types de matériaux métalliques identiques ou différents.

6. Vaporisateur selon la revendication 5, dans lequel, lorsque le premier manchon et le second manchon sont fabriqués à partir de matériaux magnétiques métalliques différents, le premier manchon a une plus grande perméabilité magnétique que le second manchon.

7. Vaporisateur selon la revendication 1, **caractérisé en ce qu'**une couche isolante entoure l'extérieur du manchon, configurée pour réduire la chaleur du manchon délivrée à l'extérieur.

8. Vaporisateur selon la revendication 1, **caractérisé en ce que** le manchon comprend un réceptacle à tabac pour recevoir les matières solides du tabac.

9. Le vaporisateur selon l'une quelconque des revendications 1 à 8, **caractérisé en ce que** le vaporisateur comprend en outre un module d'alimentation électrique, couplé à la bobine d'induction électromagnétique, et configuré pour fournir de l'énergie à la bobine d'induction électromagnétique.

10. Vaporisateur selon la revendication 9, **caractérisé en ce que** la bobine d'induction électromagnétique comprend une première interface et une deuxième interface ; la première interface et la deuxième interface sont couplées au module d'alimentation électrique.

11. Vaporisateur selon la revendication 10, **caractérisé en ce qu'**une couche isolante est appliquée sur la bobine d'induction électromagnétique, configurée pour empêcher la fuite d'électricité de la bobine d'induction électromagnétique.

12. Vaporisateur selon la revendication 11, **caractérisé en ce que** le module d'alimentation électrique comprend une interface USB, une batterie, une unité de commande, un circuit de charge, un circuit de décharge, un circuit de détection de tension, deux interrupteurs, un circuit de gestion de la batterie et un convertisseur ; la batterie est couplée au circuit de charge et au circuit de décharge, deux interrupteurs sont respectivement disposés entre la batterie et le circuit de charge, et entre la batterie et le circuit de décharge ; le circuit de charge et le circuit de décharge sont tous deux couplés avec l'interface USB ; le circuit de décharge est couplé avec le circuit de gestion de la batterie ; le circuit de gestion de la batterie est couplé avec le convertisseur ; le convertisseur est couplé avec la bobine d'induction électromagnétique, le circuit de détection de la tension est couplé avec l'interface USB ; l'unité de commande est couplée avec les deux interrupteurs et le circuit de détection de la tension.

13. Ensemble à fumer (30) cuit à basse température, comprenant un boîtier (20) et le vaporisateur selon l'une quelconque des revendications 1 à 12 ; le vaporisateur est disposé à l'intérieur du boîtier.
